# EUROPEAN PATENT APPLICATION

(11) **EP 2 452 682 A1**
(43) Date of publication of application: **16.05.2012**
(21) Application number: 12154010.8
(22) Date of filing: 01.02.2007
(51) Int. Cl.: A61K 31/47, A61K 31/472, A61K 31/506, A61P 31/18, C07D 215/56

(54) **Use of 6-(3-chloro-2-fluorobenzyl)-1-[(2s)-1-hydroxy-3-methylbutan-2-yl]-7-methoxy-4-oxo-1,4-dihydroquinoline-3-carboxylic acid or salt thereof for treating retrovirus infection**

(30) Priority: 01.02.2006 US 763900 P
(62) Divisional of application: 07713912.9
(71) Applicant: Japan Tobacco, Inc., Tokyo 105-8422 (JP)
(72) Inventor: Matsuzaki, Yuji, Osaka, 569-1125 (JP); Kano, Mitsuki, Osaka, 569-1125 (JP); Ikeda, Satoru, Osaka, 569-1125 (JP)
(74) Representative: Vossius & Partner

(57) **Abstract**

The present invention provides a use of a therapeutically effective amount of 6-(3-chloro-2-fluorobenzyl)-1-[(2S)-1-hydroxy-3-methylbutan-2-yl]-7-methoxy-4-oxo-1,4-dihydroquinoline-3-carboxylic acid (herein referred to as Compound I) or a pharmaceutically acceptable salt thereof, for the production of an agent for the treatment in a patient. The invention further provides a use of Compound I or a salt thereof for an agent for inhibition of integrase activity. Compound I or a salt thereof is also effective in inhibiting the replication of a retrovirus resistant to at least one anti-retroviral drug. In the use of the invention, Compound I or a salt thereof may be administered alone or in combination with at least one anti-retroviral drug other than Compound I or a salt thereof. The present invention also provides kits comprising Compound I or a salt thereof.

## Description

### TECHNICAL FIELD

The present invention relates to a use of 6-(3-chloro-2-fluorobenzyl)-1-[(2*S*)-1-hydroxy-3-methylbutan-2-yl]-7-methoxy-4-oxo-1,4-dihydroquinoline-3-carboxylic acid or a pharmaceutically acceptable salt thereof, for the production of agents, compositions and kits for treating a retrovirus infection.

### BACKGROUND ART

Retroviruses are RNA viruses and are generally classified into the alpharetrovirus, betaretrovirus, deltaretrovirus, epsilonretrovirus, gammaretrovirus, lentivirus, and spumavirus families. Examples of retroviruses include, but are not limited to, human immunodeficiency virus (HIV), human T-lymphotropic virus (HTLV), rous sarcoma virus (RSV), and the avian leukosis virus.

Despite the large number of retroviruses thus far isolated, all retroviruses appear similar in the organization of their genome, general virion structure, and mode of replication. The genome consists of two identical molecules of single-stranded RNA, ranging from 7 to 11 kb in length. The viral genes encode structural and enzymatic proteins in the invariable order *gag-*pol-env, although some retroviruses, such as lentiviruses, additionally encode regulatory proteins. The virion is enveloped and is about 100 nm in diameter, with envelope glycoproteins (products of the env gene) projecting out from the surface. Products of the gag gene, usually three or four, comprise the internal structural core. Included in the core are the viral enzymes encoded from the *pol* gene: the protease, reverse transcriptase, and integrase.

Replication of retroviruses occurs in two distinct steps. The first step occurs with little or no assistance from the host cell. Following the binding of viral surface glycoproteins to cellular receptors, retroviruses enter host cells by either receptor-mediated endocytosis or direct fusion of the viral and host membranes (Marsh and Helenius, Adv. Viral Res. 36:107-151, 1989). The viral core containing the two single-stranded RNA genomes are released into the cytoplasm and the viral reverse transcriptase transcribes the RNA genomes into linear doublestranded DNA (Gilboa et al., Cell 18:93-100, 1979). The linear DNA is transported into the nucleus and becomes integrated into the host genome by the action of the viral integrase (Brown et al., Proc. Natl. Acad. Sci. USA 86:2525-2529, 1989; Fujiwara and Mizuuchi, Cell 54:497-504, 1988). Integration occurs at random sites in the host chromosome and produces only one integrated copy of viral DNA per infectious viral particle. The integrated DNA is called the provirus.

In the second stage, the provirus becomes dependent on cellular functions for its transcription and translation. New viral particles are produced by the assembly of viral cores, association of the cores with the cell membrane, followed by budding from the membrane through a non-lytic mechanism.

Human Immunodeficiency Virus (HIV) is the causative agent of Acquired Immunodeficiency Syndrome (AIDS) (Barre-Sinossi et al., Science 220: 868-870, 1983). The CD4+ lymphocytes are the major target cells of HIV (Dalgleish et al., Nature 312:767-768, 1984), although HIV is also capable of infecting macrophages, neurons, and other cells (Maddon et al., Cell 47:333-48, 1986). HIV infection in a human body destroys CD4+ lymphocytes such that the body begins to lose its immune function. A person with AIDS is therefore highly vulnerable to various infections, neuronal dysfunction, tumors, and other diseases, disorders, and conditions as a result of HIV infection.

With its severe symptoms and high mortality rate, the epidemic contagion of AIDS has become one of the leading causes of death that is threatening human health. UNAIDS estimated that in 2005, 40.3 million people were living with HIV throughout the world. In 2005 alone, approximately 4.9 million new HIV infections were reported. And in 2005 alone, approximately 3.1 million people have died from AIDS. See UNAIDS/WHO "AIDS Epidemic Update: December 2005", Complete Report, p. 76-80.

At least two types of HIV have been identified: HIV-1 (Gallo et. al., Science 224:500-503, 1984) and HIV-2 (Clavel et al., Science 223:343-346, 1986). However, each has high genetic heterogeneity. HIV-1 alone exhibits at least 11 different genotypes (A-J and O subtypes) (Jonassen et al., Virol. 231:43-47, 1997).

Despite great efforts by the scientific community, there is yet no working vaccine or cure for HIV or AIDS. The complexity and genetic variability of HIV make the development of a vaccine and cure challenging (Bloom, Science 272:1888-1900, 1996).

Nonetheless, two main categories of drugs have been approved for treatment of HIV infection and AIDS: reverse transcriptase inhibitors and protease inhibitors. Both target the later stages of HIV infection-transcription and assembly of new viruses. The well-known "Cocktail Therapy" is a combination therapy using both types of inhibitors (Lafeuillade et al., J. Infect. Dis. 175:1051-55, 1997).

Drug resistance has been associated with both types of drugs. Drug resistance occurs when viruses become less sensitive to the drugs and a greater concentration of the drug is required to produce the same inhibitory effect. For reverse transcriptase inhibitors, including AZT, ddI, ddC, 3TC, and d4T, the effective inhibition concentration of the drugs has been reported as having increased several-fold to even ten-fold (Vella and Floridia, International AIDS Society USA 4(3):15, 1996). Drug resistance has been associated with all protease inhibitors presently used in AIDS treatment (Condra et al., Nature 374:569-71, 1995).

This drug-resistance is associated with a high mutation rate of HIV. In the human body, a single HIV is capable of producing 10⁸-10¹⁰ new viruses every day, with a mutation rate of 3 x 10⁵ per replication cycle. In some HIV strains, 40% of the amino acid sequences of certain proteins are altered due to mutations of their genes (Myers and Montaner, The Retroviridae Vol. 1, Plenum Press, New York, p. 51-105, 1992). Additionally, mutations in the viral protease gene are known to be the cause of drug-resistance in all currently used protease inhibitors (Condra et al., Nature 374:569-571, 1995).

Therefore, there is still a need for an effective treatment for retrovirus infections, in particular, HIV infection by drug-resistant strains. Moreover, there is a need to inhibit the increase of drug-resistant HIV in the human body.

### DISCLOSURE OF INVENTION

The present invention provides a use of a therapeutically effective amount of 6-(3-chloro-2-fluorobenzyl)-1-[(2S)-1-hydroxy-3-methylbutan-2-yl]-7-methoxy-4-oxo-1,4-dihydroquinoline-3-carboxylic acid (herein referred to as Compound I) or a salt thereof, for the production of an agent for the treatment of a retrovirus infection in a patient. It has also been discovered that Compound I or a salt thereof is effective in inhibiting the replication of a retrovirus resistant to at least one anti-retroviral drug. Compound I or a salt thereof may be administered alone or in combination with at least one anti-retroviral drug other than Compound I or a salt thereof.

The present invention provides a method for treating a human immunodeficiency virus (HIV) infection by administering to a patient a therapeutically effective amount of Compound I or a salt thereof. The Compound I or a salt thereof may be administered alone or in combination with at least one substance having anti-HIV activity other than Compound I or a salt thereof. In one embodiment of the invention, the HIV may be resistant to at least one anti-HIV drug. In another embodiment, the patient may have resistance to an anti-HIV drug. The at least one substance having anti-HIV activity and the at least one anti-HIV drug may be the same or different, and may be a protease inhibitor, a nucleoside or a non-nucleoside reverse transcriptase inhibitor, or an integrase inhibitor.

The invention also provides a method for inhibiting HIV integrase activity by administering to a patient a therapeutically effective amount of Compound I or a salt thereof. The Compound I or a salt thereof may be administered alone or in combination with at least one substance having anti-HIV activity other than Compound I or a salt thereof. Additionally, the HIV may be resistant to at least one anti-HIV drug. The at least one substance having anti-HIV activity and the at least one anti-HIV drug may be the same or different, and may be a protease inhibitor, a nucleoside or a non-nucleoside reverse transcriptase inhibitor, or an integrase inhibitor.

The invention further provides a method for inhibiting HIV integrase activity of a HIV resistant to at least one anti-HIV drug. The method comprises contacting an effective amount of Compound I or a salt thereof with an HIV resistant to at least one anti-HIV drug.

The present invention also provides a pharmaceutical composition and a kit comprising Compound I or a salt thereof and at least one substance having anti-HIV activity. The at least one substance having anti-HIV activity may be a protease inhibitor, a nucleoside or non-nucleoside reverse transcriptase inhibitor, or an integrase inhibitor.

The present invention provides an anti-retrovirus infection agent comprising a therapeutically effective amount of Compound I or a salt thereof. It has also been discovered that Compound I or a salt thereof is effective in inhibiting the replication of a retrovirus resistant to at least one anti-retroviral drug. The agent may comprise Compound I or a salt thereof alone or in combination with at least one anti-retroviral substance other than Compound I or a salt thereof.

The present invention provides an anti-HIV agent comprising a therapeutically effective amount of Compound I or a salt thereof. The agent may comprise Compound I or a salt thereof alone or in combination with at least one substance having anti-HIV activity other than Compound I or a salt thereof. In one embodiment of the invention, the HIV may be resistant to at least one anti-HIV drug. In another embodiment, the agent may be administrated to a patient who has resistance to an anti-HIV drug. The at least one substance having anti-HIV activity and the at least one anti-HIV drug may be the same or different, and may be a protease inhibitor, a nucleoside or a non-nucleoside reverse transcriptase inhibitor, or an integrase inhibitor.

The invention also provides a HIV integrase inhibitor comprising a therapeutically effective amount of Compound I or a salt thereof. The inhibitor may be comprising Compound I or a salt thereof alone or in combination with at least one substance having anti-HIV activity other than Compound I or a salt thereof. Additionally, the HIV may be resistant to at least one anti-HIV drug. The at least one substance having anti-HIV activity and the at least one anti-HIV drug may be the same or different, and may be a protease inhibitor, a nucleoside or a non-nucleoside reverse transcriptase inhibitor, or an integrase inhibitor.

Accordingly, examples of embodiments of the present invention include, and are not limited to, the following.
[1] Use of a therapeutically effective amount of (i) 6-(3-chloro-2-fluorobenzyl)-1-[(2*S*)-1-hydraxy-3-methylbutan-2-yl]-7-methoxy-4-oxo-1,4-dihydroquinoline-3-carboxylic acid, or a pharmaceutically acceptable salt thereof, for the production of an agent for the treatment of a human immunodeficiency virus (HIV) infection in a patient, wherein the HIV is resistant to at least one anti-HIV drug.
[2] The use of [1], wherein the at least one anti-HIV drug is selected from a protease inhibitor and a reverse transcriptase inhibitor.
[3] The use of [2], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[4] The use of [2], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[5] The use of [1], wherein the agent is orally administrated to a patient.
[6] Use of a therapeutically effective amount of (i) 6-(3-chloro-2-fluorobenzyl)-1-[(2*S*)-1-hydroxy-3-methylbutan-2-yl]-7-methoxy-4-oxo-1,4-dihydroquinoline-3-carboxylic acid, or a pharmaceutically acceptable salt thereof, for the production of an agent for the treatment of a human immunodeficiency virus (HIV) infection in a patient, wherein the patient has a resistance to at least one anti-HIV drug.
[7] The use of [6], wherein the at least one anti-HIV drug is selected from a protease inhibitor and a reverse transcriptase inhibitor.
[8] The use of [7], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[9] The use of [7], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[10] The use of [6], wherein the agent is orally administrated to a patient.
[11] Use of a therapeutically effective amount of (i) 6-(3-chloro-2-fluorobenzyl)-1-[(2*S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), for the production of an agent for the treatment of a HIV infection comprising in a patient, wherein the HIV is resistant to at least one anti-HIV drug.
[12] The use of [11], wherein the at least one anti-HIV drug and the at least one substance having anti-HIV activity are the same.
[13] The use of [11], wherein the at least one anti-HIV drug and the at least one substance having anti-HIV activity are different.
[14] The use of [11], wherein the at least one anti-HIV drug is selected from a protease inhibitor and a reverse transcriptase inhibitor.
[15] The use of [11], wherein the at least one substance having anti-HIV activity is selected from a protease inhibitor and a reverse transcriptase inhibitor.
[16] The use of [14] or [15], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[17] The use of [14] or [15], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[18] The use of [11], wherein (i) and (ii) are orally administrated to a patient.
[19] The use of [11], wherein (i) and (ii) are simultaneously administrated to a patient.
[20] The use of [11], wherein (i) and (ii) are sequentially administrated to a patient.
[21] Use of a therapeutically effective amount of (i) 6-(3-chloro-2-fluorobenzyl)-1-[(2*S*)-1-hydroxy-3-methylbutan-2-yl]-7-methoxy-4-oxo-1,4-dihydroquinoline-3-carboxylic acid, or a pharmaceutically acceptable salt thereof, for the production of an agent for the inhibition of HIV integrase activity in a patient, wherein the HIV is resistant to at least one anti-HIV drug.
[22] The use of [21], wherein the at least one anti-HIV drug is selected from a protease inhibitor and a reverse transcriptase inhibitor.
[23] The use of [22], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[24] The use of [22], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[25] Use of a therapeutically effective amount of (i) 6-(3-chloro-2-fluorobenzyl)-1-[(2*S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), for the production of an agent for the inhibition of HIV integrase activity in a patient, wherein the HIV is resistant to at least one anti-HIV drug.
[26] The use of [25], wherein the at least one anti-HIV drug and the at least one substance having anti-HIV activity are the same.
[27] The use of [25], wherein the at least one anti-HIV drug and the at least one substance having anti-HIV activity are different.
[28] The use of [25], wherein the at least one anti-HIV drug is selected from a protease inhibitor and a reverse transcriptase inhibitor.
[29] The use of [25], wherein the at least one substance having anti-HIV activity is selected from a protease inhibitor and a reverse transcriptase inhibitor.
[30] The use of [28] or [29], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[31] The use of [28] or [29], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[32] The use of [25], wherein (i) and (ii) are orally administrated to a patient.
[33] The use of [25], wherein (i) and (ii) are simultaneously administrated to a patient.
[34] The use of [25], wherein (i) and (ii) are sequentially administrated to a patient.
[35] Use of (i) 6-(3-chloro-2-fluorobenzyl)-1-[(2*S*)-1-hydroxy-3-methylbutan-2-yl]-7-methoxy-4-oxo-1,4-dihydroquinoline-3-carboxylic acid, or a pharmaceutically acceptable salt thereof, for inhibiting HIV integrase activity, comprising contacting an effective amount of (i) with a HIV resistant to at least one anti-HIV drug.
[36] The use of [35], wherein the at least one anti-HIV drug is selected from a protease inhibitor and a reverse transcriptase inhibitor.
[37] Use of a therapeutically effective amount of (i) 6-(3-chloro-2-fluorobenzyl)-1-[(2*S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), for the production of an agent for the treatment of a HIV infection to a patient, wherein the at least one substance having anti-HIV activity is selected from Truvada® (tenofovir + emtricitabine), elvucitabine, GW 204937, GW 678248, MK-0518, RSC 1838, V-165, C-2507, BMS 538158, and L-900564.
[38] The use of [37], wherein (i) and (ii) are orally administrated to a patient.
[39] The use of [37], wherein (i) and (ii) are simultaneously administrated to a patient.
[40] The use of [37], wherein (i) and (ii) are sequentially administrated to a patient.
[41] A pharmaceutical composition comprising (i) 6-(3-chloro-2-fluorobenzyl)-1-[(2*S*)-1-hydroxy-3-methylbutan-2-yl]-7-methoxy-4-oxo-1,4-dihydroquinoline-3-carboxylic acid, or a pharmaceutically acceptable salt thereof, (ii) at least one substance having anti-HIV activity is selected from Truvada® (tenofovir + emtricitabine), elvucitabine, GW 204937, GW 678248, MK-0518, RSC 1838, V-165, C-2507, BMS 538158, and L-900564; and (iii) a pharmaceutically acceptable carrier.
[42] A kit comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity is selected from Truvada® (tenofovir + emtricitabine), elvucitabine, GW 204937, GW 678248, MK-0518, RSC 1838, V-165, C-2507, BMS 538158, and L-900564.
[43] A kit comprising the pharmaceutical composition of [41].
[44] The kit of [42], wherein (i) and (ii) are simultaneously administrated to a patient.
[45] The kit of [42], wherein (i) and (ii) are sequentially administrated to a patient.
[46] An anti-HIV agent comprising (i) 6-(3-chloro-2-fluorobenzyl)-1-[(2S)-1-hydroxy-3-methylbutan-2-yl]-7-methoxy-4-oxo-1,4-dihydroquinoline-3-carboxylic acid, or a pharmaceutically acceptable salt thereof, wherein the HIV is resistant to at least one anti-HIV drug.
[47] The agent of [46], wherein the at least one anti-HIV drug is selected from a protease inhibitor and a reverse transcriptase inhibitor.
[48] The agent of [47], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[49] The agent of [47], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[50] The agent of [46], which is orally administrated to a patient.
[51] An anti-HIV agent comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, wherein a patient to whom is administrated the agent has a resistance to at least one anti-HIV drug.
[52] The agent of [51], wherein the at least one anti-HIV drug is selected from a protease inhibitor and a reverse transcriptase inhibitor.
[53] The agent of [52], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[54] The agent of [52], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[55] The agent of [51], which is orally administrated to a patient.
[56] An anti-HIV agent comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti--HIV activity other than (i), wherein the HIV is resistant to at least one anti-HIV drug.
[57] The agent of [56], wherein the at least one anti-HIV drug and the at least one substance having anti-HIV activity are the same.
[58] The agent of [56], wherein the at least one anti-HIV drug and the at least one substance having anti-HIV activity are different.
[59] The agent of [56], wherein the at least one anti-HIV drug is selected from a protease inhibitor and a reverse transcriptase inhibitor.
[60] The agent of [56], wherein the at least one substance having anti-HIV activity is selected from a protease inhibitor and a reverse transcriptase inhibitor.
[61] The agent of [59] or [60], wherein the protease inhibitor is selected from Crixivan® (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[62] The agent of [59] or [60], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[63] The agent of [56], wherein (i) and (ii) are administrated orally to a patient.
[64] The agent of [56], wherein (i) and (ii) are administrated simultaneously to a patient.
[65] The agent of [56], wherein (i) and (ii) are administrated sequentially to a patient.
[66] A HIV integrase inhititor comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, wherein the HIV is resistant to at least one anti-HIV drug.
[67] The inhibitor of [66], wherein the at least one anti-HIV drug is selected from a protease inhibitor and a reverse transcriptase inhibitor.
[68] The inhibitor of [66], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Propei^{®} (amprenavir or APV), and Reyataz^{®} (atazanavir or ATV).
[69] The inhibitor of [67], wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[70] A HIV integrase inhititor comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), wherein the HIV is resistant to at least one anti-HIV drug.
[71] The inhibitor of [70], wherein the at least one anti-HIV drug and the at least one substance having anti-HIV activity are the same.
[72] The inhibitor of [70], wherein the at least one anti-HIV drug and the at least one substance having anti-HIV activity are different.
[73] The inhibitor of [70], wherein the at least one anti-HIV drug is selected from a protease inhibitor and a reverse transcriptase inhibitor.
[74] The inhibitor of [70], wherein the at least one substance having anti-HIV activity is selected from a protease inhibitor and a reverse transcriptase inhibitor.
[75] The inhibitor of [73] or [74], wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).
[76] The inhibitor of [73] or [74], wherein the reverse transcriptase inhibitor is selected from Retrovir^{®} (zidovudine or AZT), Epivir^{®} (lamivudine or 3TC), Zerit^{®} (sanilvudine or d4T), Videx® (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).
[77] The inhibitor of [70], wherein (i) and (ii) are administrated orally to a patient.
[78] The inhibitor of [70], wherein (i) and (ii) are administrated simultaneously to a patient.
[79] The inhibitor of [70], wherein (i) and (ii) are administrated sequentially to a patient.
[80] A HIV integrase inhititor comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, in combination with a HIV resistant, wherein HIV is resistant to at least one anti-HIV drug.
[81] The inhibitor of [80], wherein the at least one anti-HIV drug is selected from a protease inhibitor and a reverse transcriptase inhibitor.
[82] An anti-HIV agent comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), wherein at least one substance is selected from Truvada® (tenofovir + emtricitabine), elvucitabine, GW 204937, GW 678248, MK-0518, RSC 1838, V-165, C-2507, BMS 538158, and L-900564; and (iii) a pharmaceutically acceptable carrier.
[83] The agent of [82], wherein (i) and (ii) are administrated orally to a patient.
[84] The agent of [82], wherein (i) and (ii) are administrated simultaneously to a patient.
[85] The agent of [82], wherein (i) and (ii) are administrated sequentially to a patient.

Compound I has the following structural formula:

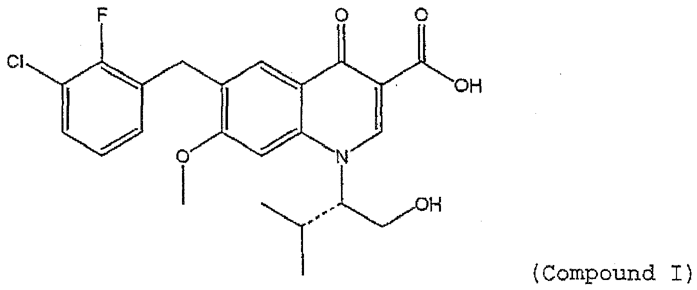

Compound I is described in U.S. Application No. 10/492,833, filed November 20, 2003 (U.S. Patent Application Publication No. 2005/0239819), and in U.S. Application No. 11/133,463, filed May 20, 2005 (U.S. Patent Application Publication No. 2005/0288326), which are herein incorporated by reference in their entirety. Compound I exists in at least three different crystal forms. Crystal forms I, II and III are described in WO 05/113508, which is herein incorporated by reference in its entirety. These three forms are distinguishable by differential scanning calorimeter (DSC) and X-ray powder diffractometer (XRD). Any one of the crystal forms may be used in the present invention. In an embodiment of the invention, crystal form II or III, or a mixed crystal thereof, is administered to a patient.

While the administration of Compound I or a salt thereof is a particular embodiment of the invention, the invention also contemplates the administration of other compounds that will yield Compound I, *e.g.*, prodrugs of Compound I. Such prodrugs, for example, may include compounds that have protecting groups, but that still result in the formation of Compound I in the body of a patient (*i.e.*, *in vivo).* Carboxylic acid-protecting groups include, for example, alkyl esters and benzyl esters, which may be removed by an acid or base and hydrogenolysis, respectively. Moreover, a compound having any organic residue that may be dissociated *in vivo* to yield Compound I may be administered according to the method of the invention. Thus, the invention also contemplates the administration of Compound I' (wherein R' signifies an organic residue) so as to yield Compound I.

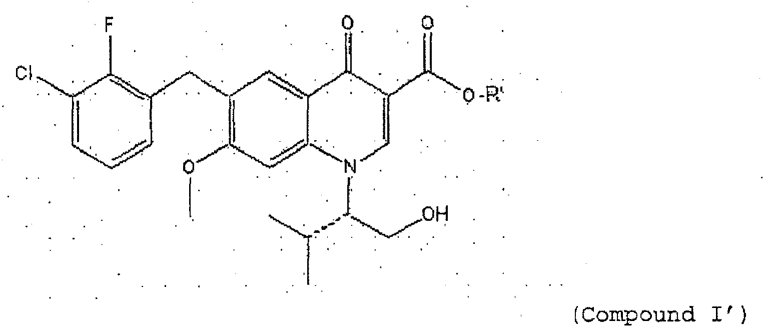

The present invention further contemplates the administration of a pharmaceutically acceptable salt of Compound I. For example, a pharmaceutically acceptable salt of Compound I may be obtained by reacting Compound I with: an inorganic acid such as hydrochloric acid, sulfuric acid, phosphoric acid, hydrobromic acid, and the like; an organic acid such as oxalic acid, malonic acid, citric acid, fumaric acid, lactic acid, malic acid, succinic acid, tartaric acid, acetic acid, trifluoroacetic acid, gluconic acid, ascorbic acid, methylsulfonic acid, benzylsulfonic acid, and the like; an inorganic base such as sodium hydroxide, potassium hydroxide, calcium hydroxide, magnesium hydroxide, ammonium hydroxide, and the like; an organic base such as methylamine, diethylamine, triethylamine, triethanolamine, ethylenediamine, tris(hydroxymethyl)methylamine, guanidine, choline, cinchonine, and the like; or an amino acid such as lysine, arginine, alanine, and the like. The present invention encompasses water-containing products and solvates, such as hydrates, of Compound I, and the like. In addition, the terms "or a salt thereof" and "or a pharmaceutically acceptable salt thereof" used herein are interchangeable.

Therefore, as used herein, "administering ... Compound I" or "administering ... 6-(3-chloro-2-fluorobenzyl)-1-[(*2S*)-1-hydroxy-3-methylbutan-2-yl]-7-methoxy-4-oxo-1,4-dihydroquinoline-3-carboxylic acid" refers to the administration of any form of Compound I that provides Compound I, *in vivo.*

The terms "anti-retroviral drug" and "substance having anti-retroviral activity" as used herein are interchangable and refer to any agent such as a chemotherapeutic, peptide, antibody, antisense, ribozyme, vaccine, immunostimulants such as interferon, a nucleoside or non-nucleoside reverse transcriptase inhibitor, protease inhibitor, integrase inhibitor, inhibitor of binding between a host cell receptor (e.g., CD4, CXCR4, CCR5) and a retrovirus, or any combination thereof, that is capable of inhibiting retrovirus replication or cytopathogenicity. Thus, an "anti-HIV drug" or a "substance having anti-HIV activity" as used herein refers to an anti-retroviral drug that is capable of inhibiting HIV replication or cytopathogenicity. As used herein, "inhibiting" refers to the decrease or cessation of at least one activity or characteristic associated with a virus, protein, enzyme, or any other compound.

Examples of a HIV reverse transcriptase inhibitor include, but are not limited to, Retrovir^{®} (zidovudine or AZT), Epivir^{®} (lamivudine or 3TC), Zerit^{®} (sanilvudine or d4T), Videx^{®} (didanosine or ddI), Hivid^{®} (zalcitabine), Ziagen^{®} (abacavir sulfate or ABC), Viramune^{®} (nevirapine or NVP), Stocrin^{®} (efavirenz or EFV), Rescriptor^{®} (delavirdine mesylate or DLV), Combivir^{®} (zidovudine+lamivudine), Trizivir^{®} (abacavir sulfate+lamivudine+zidovudine), coactinon^{®} (emivirine), Phosphonovir^{®}, Coviracil^{®}, alovudine (3'-fluora-3'-deoxythymidine), Thiovir (thiophosphonoformic acid), Capravirin (5-[(3,5-dichlorophenyl)thio]-4-isopropyl-1-(4-pyridylmethyl)imidazole-2-methanol carbamic acid), Tenofovir (PMPA or TFV), Tenofovir disoproxil fumarate ((R)-[[2-(6-amino-9H-purin-9-yl)-1-methylethoxy]methyl]phosphonic acid bis (isopropoxycarbonyloxymethyl) ester fumarate), DPC-083 ((4S)-6-chloro-4-[(1E)-cyclopropylethenyl]-3,4-dihydro-4-trifluoromethyl-2(1H)-quinazolinone), DPC-961 ((4S)-6-chloro-4-(cyclopropylethynyl)-3,4-dihydro-4-(trifluoromethyl)-2(1H)-quinazolinone), DAPD ((-)-β-D-2,6-diaminopurine dioxolane),Immunocal, MSK-055, MSA-254, MSH-143, NV-01, TMC-120, DPC-817,GS-7340, TMC-125, SPD-754, D-A4FC, capravirine, UC-781, emtricitabine, alovudine, Phosphazid, UC-781, BCH-10618, DPC-083, Etravirine, BCH-13520, MIV-210,abacavir sulfate/lamivudine, GS-7340, GW-5634, GW-695634, Truvada^{®} (tenofovir + emtricitabine), elvucitabine, GW 204937, and GW 678248.

Examples of a HIV protease inhibitor include, but are not limited to, Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), Kaletra^{®} (ritonavir+lopinavir), mozenavir dimesylate ([4R-(4α,5α,6β)]-1-3-bis[(3-aminophenyl)methyl]hexahydro-5,6-dihydroxy-4,7-bis(phenylmethyl)-2H-1,3-diazepin-2-one dimethanesulfonate), tipranavir (TPV or 3'-[(1R)-1-[(6R)-5,6-dihydro-4-hydroxy-2-oxo-6-phenylethyl-6-propyl-2H-pyran-3-yl]propyl]-5-(trifluoromethyl)-2-pyridinesulfonamide), lasinavir (N-[5(S)-(tert-butoxycarbonylamino)-4(S)-hydroxy-6-phenyl-2(R)-(2,3,4-trimethoxybenzyl)hexanoyl]-L-valine 2-methoxyethylenamide), KNI-272 ((R)-N-tert-butyl-3-[(2S,3S)-2-hydroxy-3-N-[(R)-2-N-(isoquinolin-5-yloxyacetyl)amino-3-methylthiopropanoyl]amino-4-phenylbutanoyl]-5,5-dimethyl-1,3-thiazolidine-4-carboxamide), GW-433908, TMC-126, DPC-681, buckminsterfullerene, MK-944A (MK944 (N-(2(R)-hydroxy-1(S)-indanyl)-2(R)-phenylmethyl-4(S)-hydroxy-5-[4-(2-benzo[b]furanylmethyl)-2(S)-(tert-butylcarbamoyl)piperazin-1-yl]pentanamide)+indinavir sulfate), JE-2147 ([2(S)-oxo-4-phenylmethyl-3(S)-[(2-methyl-3-oxy)phenylcarbonylamino]-1-oxabutyl]-4-[(2-methylphenyl)methylamino]carbonyl-4(R)-5,5-dimethyl-1,3-thiazole), BMS-232632 ((3S,8S,9S,12S)-3,12-bis(1,1-dimethylethyl)-8-hydroxy-4,11-dioxo-9-(phenylmethyl)-6-[[4-(2-pyridinyl)phenyl]methyl]-2,5,6,10,13-pentaazatetradecanedicarboxylic acid dimethyl ester), DMP-850 ((4R,5S,6S,7R)-1-(3-amino-1H-indazol-5-ylmethyl)-4,7-dibenzyl-3-butyl-5,6-dihydroxyperhydro-1,3-diazepin-2-one), DMP-851, RO-0334649, Nar-DG-35, R-944, VX-385, TMC-114, Fosamprenavir sodium, Fosamprenavir calcium, Darunavir, GW-0385, R-944, RO-033-4649, AG-1859, and Reyataz® (atazanavir; ATV).

Examples of an HIV integrase inhibitor include, but are not limited to, S-1360, L-870810, MK-0518, RSC 1838, V-165, C-2507, BMS 538158, and L-900564.

Other anti-HIV drugs include a DNA polymerase inhibitor or DNA synthesis inhibitor, exemplified by, but not limited to, Foscavir^{®}, ACH-126443 (L-2',3'-didehydro-dideoxy-5-fluorocytidine), entecavir ((1S,3S,4S)-9-[4-hydroxy-3-(hydroxymethyl)-2-methylenecyclopentyl]guanine), calanolide A ([10R-(10α,11β,12α)]-11,12-dihydro-12-hydroxy-6,6,10,11-tetramethyl-4-propyl-2H,6H,10H-benzo[1,2-b:3,4-b':5,6-b"]tripyran-2-one), calanolide B, NSC-674447 (1,1'-azobisformamide), Iscador (viscum album extract), and Rubutecan.

An HIV antisense drug is exemplified by, but not limited to, HGTV-43 and GEM-92. An anti-HIV antibody is exemplified by, but not limited to, NM-01, PRO-367, KD-247, Cytolin®, TNX-355 (CD4 antibody), AGT-1, PRO-140 (CCR5 antibody), and Anti-CTLA-4 Mab. A HIV vaccine is exemplified by, but not limited to, ALVAC^{®}, AIDSVAX^{®}, Remune^{®}, HIV gp41 vaccine, HIV gp120 vaccine, HIV gp140 vaccine, HIV gp160 vaccine, HIV p17 vaccine, HIV p24 vaccine, HIV p55 vaccine, AlphaVax Vector System, canarypox gp160 vaccine, AntiTat, MVA-F6 Nef vaccine, HIV rev vaccine, C4-V3 peptide, p2249f, VIR-201, HGP-30W, TBC-3B, PARTICLE-3B, and Antiferon (interferon-α vaccine). An interferon or interferon agonist is exemplified by, but not limited to, Sumiferon^{®}, MultiFeron^{®}, interferon-τ, Reticulose, Human leukocyte interferon alpha. A CCR5 antagonist is exemplified by, but not limited to, SCH-351125. An agent acting on HIV p24 is exemplified by, but not limited to, GPG-NH2 (glycyl-prolyl-glycinamide), a HIV fusion inhibitor is exemplified by, but not limited to, FP-21399 (1,4-bis[3-[(2,4-dichlorophenyl)carbonylamino]-2-oxo-5,8-disodium sulfonyl]naphthyl-2,5-dimethoxyphenyl-1,4-dihydrazone), T-1249, Synthetic Polymeric Construction No 3, pentafuside, FP-21399, PRO-542, and Enfuvirtide. An IL-2 agonist or antagonist is exemplified by, but not limited to, interleukin-2, Imunace^{®}, Proleukin^{®}, Multikine^{®}, Ontak^{®}, a TNF-α antagonist is exemplified by, but not limited to, Thalomid^{®} (thalidomide), Remicade^{®} (infliximab), and curdlan sulfate. A α-glucosidase inhibitor may be Bucast^{®}. A purine nucleoside phosphorylase inhibitor is exemplified by, but not limited to, peldesine (2-amino-4-oxo-3H,5H-7-[(3-pyridyl)methyl]pyrrolo[3,2-d]pyrimidine), an apoptosis agonist or inhibitor is exemplified by, but not limited to, Arkin Z^{®}, Panavir^{®}, and Coenzyme Q10 (2-deca(3-methyl-2-butenylene)-5,6-dimethoxy-3-methyl-p-benzoquinone), a cholinesterase inhibitor is exemplified by, but not limited to, Cognex^{®}, and an immunomodulator is exemplified by, but not limited to, Imunox^{®}, Prokine^{®}, Met-enkephalin (6-de-L-arginine-7-de-L-arginine-8-de-L-valinamide-adrenorphin), WF-10 (10-fold dilute tetrachlorodecaoxide solution), Perthon, PRO-542, SCH-D, UK-427857, AMD-070, and AK-602.

In addition, Neurotropin^{®}, Lidakol^{®}, Ancer 20^{®}, Ampligen^{®}, Anticort^{®}, Inactivin^{®}, PRO-2000, Rev M10 gene, HIV specific cytotoxic T cell (CTL immunotherapy, ACTG protocol 080 therapy, CD4-ζ gene therapy), SCA binding protein, RBC-CD4 complex, Motexafin gadolinium, GEM-92, CNI-1493, (±)-FTC, Ushercell, D2S, BufferGel^{®}, VivaGel^{®}, Glyminox vaginal gel, sodium lauryl sulfate, 2F5, 2F5/2G12, VRX-496, Ad5gag2, BG-777, IGIV-C, and BILR-255 are also examples of anti-HIV drugs.

Specific combinations of Compound I or a salt thereof with at least one anti-retroviral drug other than Compound I or a salt thereof include a combination of Compound I or a salt thereof with Efavirenz, Tenofovir, Emtricitabine, Indinavir, Nelfinavir, Atanazavir, Ritonavir+Indinavir, Ritonavir+Lopinavir, Ritonavir+Saquinavir, Didanosine+Lamivudine, Zidovudine+Didanosine, Stavudine+Didanosine, Zidovudine+Lamivudine, Stavudine+Lamivudine, Emtriva, Tenofovir + Emtricitabine, Elvucitabine, GW 204937, GW 678248, MK-0518, RSC 1838, V-165, C-2507, BMS 538158, and L-900564 (combinations are discussed further in Guidelines for the Use of Antiretroviral Agents in HIV-Infected Adults and Adolescents. Aug. 13, 2001).

An embodiment of the invention provides a combination of Compound I or a salt thereof with one anti-retroviral drug other than Compound I or a salt thereof, such as Efavirenz, Indinavir, Nelfinavir, Tenofovir, Emtricitabine, Zidovudine, Lamivudine, Elvucitabine, GW 204937, GW 678248, MK-0518, RSC 1838, V-165, C-2507, BMS 538158, and L-900564. Another embodiment of the invention provides a combination of Compound I or a salt thereof with two anti-retroviral drugs other than Compound I or a salt thereof, such as Zidovudine+Lamivudine, Tenofovir+Lamivudine, Tenofovir+Zidovudine, Tenofovir+Efavirenz, Tenofovir+Nelfinavir, Tenofovir+Indinavir, Tenofovir+Emtricitabine, Emtricitabine+Lamivudine, Emtricitabine+Zidovudine, Emtricitabine+Efavirenz, Emtricitabine+Nelfinavir, Emtricitabine+Indinavir, Nelfinavir+Lamivudine, Nelfinavir+Zidovudine, Nelfinavir+Efavirenz, Nelfinavir+Indinavir, Efavirenz+Lamivudine, Efavirenz+Zidovudine, Efavirenz+Indinavir, Efavirenz+Elvucitabine, Efavirenz+GW 204937, Efavirenz+GW 678248, Efavirenz+MK-0518, Efavirenz+RSC 1838, Efavirenz+V-165, Indinavir+Elvucitabine, Indinavir+GW 204937, Indinavir+GW 678248, Indinavir+MK-0518, Indinavir+RSC 1838, Indinavir+V-165, Nelfinavir+Elvucitabine, Nelfinavir+GW 204937, Nelfinavir+GW 678248, Nelfinavir+MK-0518, Nelfinavir+RSC 1838, Nelfinavir+V-165, Tenofovir+Elvucitabine, Tenofovir+GW 204937, Tenofovir+GW 678248, Tenofovir+MK-0518, Tenofovir+RSC 1838, Tenofovir+V-165, Emtricitabine+Elvucitabine, Emtricitabine+GW 204937, Emtricitabine+GW 678248, Emtricitabine+MK-0518, Emtricitabine+RSC 1838, Emtricitabine+V-165, Zidovudine+Elvucitabine, Zidovudine+GW 204937, Zidovudine+GW 678248, Zidovudine+MK-0518, Zidovudine+RSC 1838, Zidovudine+V-165, Lamivudine+Elvucitabine, Lamivudine+GW 204937, Lamivudine+GW 678248, Lamivudine+MK-0518, Lamivudine+RSC 1838, Lamivudine+V-165, Elvucitabine+GW 204937, Elvucitabine+GW 678248, Elvucitabine+MK-0518, Elvucitabine+RSC 1838, Elvucitabine+V-165, GW 204937+GW 678248, GW 204937+MK-0518, GW 204937+RSC 1838, GW 204937+V-165, GW 678248+MK-0518, GW 678248+RSC 1838, GW 678248+V-165, MK-0518+RSC 1838, MK-0518+V-165, and RSC 1838+V-165.

An alteration in viral drug sensitivity may be determined by a change in susceptibility of a viral strain to the drug. Susceptibilities are generally expressed as ratios of EC₅₀ or EC₉₀ values (concentration of the drug at which 50% or 90%, respectively, of the viral population is inhibited from replicating) of a viral strain under investigation compared to the wild type strain. Hence, the susceptibility of a viral strain towards a certain drug may be expressed as a fold change in susceptibility, wherein the fold change is derived from the ratio of, for instance, the EC₅₀ values of a mutant viral strain compared to the wild type EC₅₀ values. In particular, the susceptibility of a viral strain or population may also be expressed as resistance of a viral strain, wherein the result is indicated as a fold increase in EC₅₀ as compared to wild type EC₅₀.

Resistance of a viral strain to antiretroviral drugs may also be determined by genotype by identifying mutations in the retroviral genome that are known or shown to correlate with resistance. Mutations in a retrovirus may be detected by first amplifying the viral genomic sequence by the polymerase chain reaction (PCR), sequencing the viral genome, and comparing the sequence with a wild-type viral genome. To determine whether the mutation identified correlates with drug resistance, the mutation may be compared to a database of mutations known to correlate with drug resistance, such as one that can be found at http://hivdb.stanford.edu. Known mutations in the HIV genome associated with drug resistance is also provided, for example, in D'Aquila et al., Topics in HIV Medicine 11:92-96 (2003).

Alternatively, the cytopathogenicity and/or replication of the retrovirus harboring the mutation(s) may be compared to that of a wild-type virus (or a reference virus) in the presence of one or more anti-retroviral drugs. For instance, the relevant coding regions of, for example, the protease, reverse transcriptase, or integrase, are obtained from patient samples, reverse transcribed and amplified by PCR. The relevant coding regions are inserted into viral constructs to create chimeric viruses. The cytotopathogenicity and replication of these chimeric viruses is assessed in the presence of anti-retroviral drugs and compared with that of a wild-type or reference virus.

The susceptibility of a retrovirus to a drug may be tested by determining the cytopathogenicity of the retrovirus to cells. In the context of this invention, the cytopathogenic effect means the viability of the cells in culture in the presence of a retrovirus. The cells may be any cell that is capable of producing new infectious virus particles and may include, but are not limited to, peripheral blood mononuclear cells (PBMC), T cells, monocytes, macrophages, dendritic cells, Langerhans cells, hematopoetic stem cells or precursor cells, MT4 cells and PM-1 cells. MT4 is a CD4⁺ T-cell line containing the CXCR4 coreceptor. The PM-1 cell line expresses both the CXCR4 and CCR5 co-receptors. The cytopathogenicity may, for example, be monitored by the presence of any reporter molecule including reporter genes. A reporter gene is defined as a gene whose product has reporting capabilities. Suitable reporter molecules include, but are not limited to, tetrazolium salts, green fluorescent proteins, beta-galactosidase, chloramfenicol transferase, alkaline phophatase, and luciferase. Several methods of cytopathogenic testing including phenotypic testing are described in the literature (Kellam and Larder, Antimicrob. Agents Chemotherap. 1994, 38:23-30, 1994, Hertogs et al. Antimicrob. Agents Chemotherap. 42:269-276, 1998; Pauwels et al. J. Virol Methods 20:309-321, 1988).

The susceptibility of a retrovirus to a drug may also be determined by the replicative capacity of the virus in the presence of at least one anti-retroviral drug, relative to the replicative capacity of a wild-type retrovirus. Replicative capacity means the ability of the virus to grow under culturing conditions. The methods for determining the susceptibility or a retrovirus to a drug may also be useful for designing a treatment regimen for an HIV-infected patient. For example, a method may comprise determining the replicative capacity of a clinical isolate of a patient and using said replicative capacity to determine an appropriate drug regime for the patient. One approach is the Antivirogram® assay (Virco Lab Inc., USA, NJ).

Therefore, the term "resistant to at least one anti-retroviral drug" or "resistant to at least one anti-HIV drug" as used herein refers to the condition where the fold increase is greater than 1 and wherein the retrovirus contains at least one mutation correlating with the fold increase. As used herein, a retrovirus "highly resistant to at least one anti-retroviral drug" or "highly resistant to at least one anti-HIV drug" refers to the condition where the fold increase is equal to or greater than 100 and contains at least one mutation correlating with the fold increase. Similarly, a retrovirus "moderately resistant to at least one anti-retroviral drug" or "moderately resistant to at least one anti-HIV drug" refers to the condition where the fold increase is equal to or greater than 50 but less than 100 and contains a mutation correlating with the fold increase. A retrovirus "slightly resistant to at least one anti-retroviral drug" or "slightly resistant to at least one anti-HIV drug" refers to the condition where the fold increase is greater than 1 but less than 50 and at least one mutation correlating with the fold increase. A fold increase equal to or less than 1 or no mutations correlating with a fold increase suggests that no resistance has yet developed to the anti-retroviral drug.

The term "patient", as used herein, refers to a mammal, such as human, mouse, rat, hamster, rabbit, cat, dog, bovine, sheep, and swine. Additionally, as used herein, the term "patient has a resistance to an anti-HIV drug" refers to a patient infected with HIV who requires a greater therapeutically effective amount of the anti-HIV drug than the standard or recommended amount for the given anti-HIV drug. Thus, for a commercially-available anti-HIV drug, a patient resistant to the anti-HIV drug may require a greater amount of the anti-HIV drug in order to obtain a measurable decrease in HIV load or activity than the recommended dose.

The term "therapeutically effective amount" refers to an amount of Compound I or a salt thereof, an anti-retroviral drug, or combinations thereof, that is effective in treating the named disease, disorder, or condition. Thus, for example, an amount effective to inhibit integrase activity is an amount that results in a measurable decrease in viral integrase activity, as measured using an assay and end-point appropriate for that measurement.

While the dose varies depending on factors such as age, body weight, symptom, treatment effect, administration method, Compound I or a salt thereof is generally administered at about 1 mg to about 2000 mg per administration for an adult, given once to several times a day orally or by any other method, such as by parenteral intravenous injection. The effective dosage of other anti-retroviral drugs for administration in combination with Compound I or a salt thereof may be readily available in published sources to one of ordinary skill in the art. The present invention contemplates the use of the clinically intended effective dosage schedules for other anti-retroviral drugs.

In'a combination therapy, Compound I or a salt thereof may be administered in dosages ranging from about 1 mg to about 2000 mg per administration. Thus, for example, single administrations of Compound I in the combination therapy may be 1 mg, 2 mg, 5 mg, 10 mg, 15 mg, 20 mg, 50 mg, 100 mg, 200 mg, 300 mg, 400 mg, 500 mg, 600 mg, 700 mg, 800 mg, 900 mg, or 1000 mg, per administration. For zidovudine (AZT), for example, a recommended oral dose of zidovudine for adults for treating HIV infection is 100 mg every 4 hours. Also for example, the recommended dose for a standard intravenous administration of zidovudine is about 1 to about 2 mg/kg every 4 hours. Other anti-retroviral drugs may be administered at doses consistent with the recognized recommended administration schedules. Examples of such dosages may be any dosage ranging from 1 mg to 2000 mg per administration. For example, single administrations of the at least one anti-HIV drug may be 20 mg, 50 mg, 100 mg, 200 mg, 300 mg, 400 mg, 500 mg, 600 mg, 700 mg, 800 mg, 900 mg, 1000 mg, 1200 mg, 1400 mg, 1600 mg, 1800 mg, or 2000 mg per administration.

An embodiment of the present invention also contemplates dosages of the anti-retroviral drugs that are below the clinically intended effective dosage schedules. In some embodiments of the present invention, the beneficial effects and efficacy of Compound I or a salt thereof may permit administration of effective doses of at least one anti-retroviral agent that are lower than the clinically intended effective dosage or the recommended dosage. For example, an embodiment of the present invention contemplates the use of doses for other anti-retroviral drugs that range from, for example, 5% to 99%, such as 5%, 10%, 20%, 30%, 50%, 75%, or 90%, of the lowest clinically intended effective dosage for the anti-retroviral drug.

While it is possible for Compound I or a salt thereof and/or at least one anti-retroviral drug to be administered as a raw compound, it is preferably administered as a pharmaceutical composition. A "pharmaceutical composition comprising Compound I" refers to a pharmaceutical composition comprising any form of Compound I, including Compound I, or a pharmaceutically acceptable salt thereof, with one or more pharmaceutically acceptable carriers or excipients and optionally other therapeutic agents and/or components. Similarly, "a pharmaceutical composition comprising at least one substance having anti-retroviral activity" refers to a pharmaceutical composition comprising any form of the at least one substance having anti-retroviral activity and any of its active forms, that provides the active form of the at least one substance having anti-retroviral activity, or a pharmaceutically acceptable salt thereof, with one or more pharmaceutically acceptable carriers or excipients and optionally other therapeutic agents and/or components. A pharmaceutical composition may comprise a combination of Compound I or a salt thereof and at least one anti-retroviral drug having anti-retroviral activity.

The salt, carrier, or excipient must be acceptable in the sense of being compatible with the other ingredients and not deleterious to the recipient thereof. Examples of carriers or excipients for oral administration include cornstarch, lactose, magnesium stearate, talc, microcrystalline cellulose, stearic acid, povidone, crospovidone, dibasic calcium phosphate, sodium starch glycolate, hydroxypropyl cellulose (e.g., low substituted hydroxypropyl cellulose), hydroxypropylmethyl cellulose (e.g., hydroxypropylmethyl cellulose 2910), and sodium lauryl sulfate.

The pharmaceutical compositions may be prepared by any suitable method, such as those methods well known in the art of pharmacy, for example, methods such as those described in Gennaro et al., Remington's Pharmaceutical Sciences (18th ed., Mack Publishing Co., 1990), especially Part 8: Pharmaceutical Preparations and their Manufacture. Such methods include the step of bringing into association Compound I or a salt thereof, or Compound I or a salt thereof and at least one anti-retroviral drug with the carrier or excipient and optionally one or more accessory ingredients. Such accessory ingredients include those conventional in the art, such as, fillers, binders, diluents, disintegrants, lubricants, colorants, flavoring agents, and wetting agents.

The pharmaceutical compositions may provide controlled, slow release, or sustained release of the Compound I or a salt thereof and anti-retroviral drugs over a period of time. The controlled, slow release, or sustained release may maintain Compound I or a salt thereof and anti-retroviral drugs in the bloodstream of the patient for a longer period of time than with conventional formulations. Pharmaceutical compositions include, but are not limited to, coated tablets, pellets, and capsules, and dispersions in a medium that is insoluble in physiologic fluids or where the release of the therapeutic compound follows degradation of the pharmaceutical composition due to mechanical, chemical, or enzymatic activity.

The pharmaceutical composition of the invention may be, for example, in the form of a pill, capsule, or tablet, each containing a predetermined amount of Compound I or a salt thereof and/or an anti-retroviral drug and preferably coated for ease of swallowing, in the form of a powder or granules, or in the form of a solution or suspension. In an embodiment of the invention, the pharmaceutical composition is in the form of a tablet.

For oral administration, fine powders or granules may contain diluting, dispersing, and or surface active agents and may be present, for example, in water or in a syrup, in capsules or sachets in the dry state, or in a nonaqueous solution or suspension wherein suspending agents may be included, or in tablets wherein binders and lubricants may be included. The pharmaceutical composition may also include additional components such as sweeteners, flavoring agents, preservatives (e.g., antimicrobial preservatives), suspending agents, thickening agents, and/or emulsifying agents.

When administered in the form of a liquid solution or suspension, the formulation may contain purified water. Optional components in the liquid solution or suspension include suitable sweeteners, flavoring agents, preservatives (e.g., antimicrobial preservatives), buffering agents, solvents, and mixtures thereof. A component of the formulation may serve more than one function. For example, a suitable buffering agent also may act as a flavoring agent as well as a sweetener.

Suitable sweeteners include, for example, saccharin sodium, sucrose, and mannitol. A mixture of two or more sweeteners may be used. The sweetener or mixtures thereof are typically present in an amount of from about 0.001% to about 70% by weight of the total composition. Suitable flavoring agents may be present in the pharmaceutical composition to provide a cherry flavor, cotton candy flavor, or other suitable flavor to make the pharmaceutical composition easier for a patient to ingest. The flavoring agent or mixtures thereof are typically present in an amount of about 0.0001% to about 5% by weight of the total composition.

Suitable preservatives include, for example, methylparaben, propylparaben, sodium benzoate, and benzalkonium chloride. A mixture of two or more preservatives may be used. The preservative or mixtures thereof are typically present in an amount of about 0.0001% to about 2% by weight of the total composition.

Suitable buffering agents include, for example, citric acid, sodium citrate, phosphoric acid, potassium phosphate, and various other acids and salts. A mixture of two or more buffering agents may be used. The buffering agent or mixtures thereof are typically present in an amount of about 0.001% to about 4% by weight of the total composition.

Suitable solvents for a liquid solution or suspension include, for example, sorbital, glycerin, propylene glycol, and water. A mixture of two or more solvents may be used. The solvent or solvent system is typically present in an amount of about 1% to about 90% by weight of the total composition.

The pharmaceutical composition may be co-administered with adjuvants. For example, nonionic surfactants such as polyoxyethylene oleyl ether and n-hexadecyl polyethylene ether may be administered with or incorporated into the pharmaceutical composition to artificially increase the permeability of the intestinal walls. Enzymatic inhibitors may also be administered with or incorporated into the pharmaceutical composition.

The combination of Compound I or a salt thereof and an anti-retroviral drug may be formulated as separate compositions which are administered at substantially the same time. Alternatively, Compound I or a salt thereof and the anti-retroviral drug may be administered to the host at different times such that only one or two active agents at a time are at a therapeutically effective amount in the host. In another embodiment of the invention, the combination of Compound I or a salt thereof and the anti-retroviral drug is formulated as a single composition such that all of the active agents are administered at a therapeutically effective amount to the host in each dose. Accordingly, Compound I or a salt thereof and other anti-retroviral agents may be administered to an individual either sequentially or simultaneously.

The compositions and methods of the present invention may also be used to inhibit HIV replication or HIV integrase activity. For example, an effective amount of Compound I or a salt thereof may be contacted with HIV or HIV resistant to at least one anti-HIV drug. In this context, "effective amount" refers to an amount that results in a measurable decrease in viral replication or integrase activity, as measured using an assay and end-point appropriate for that measurement. For instance, an effective amount to inhibit viral replication or integrase activity *in vivo* is that amount that, following administration, yields a detectable improvement in the symptoms associated with the given disease or condition under treatment. In an in vitro assay, viral replication or viral integrase inhibition may be measured more directly, for example, by a decrease in virus-induced cytotoxicity.

The compositions and methods of the present invention may further be used to identify additional anti-retroviral drugs that may be beneficial for combination therapy with Compound I or a salt thereof for treating HIV infections. For example, a test compound may be added to an HIV infected cell culture in combination with Compound I or a salt thereof, or Compound I or a salt thereof plus at least one other anti-HIV drug and the replication of the retrovirus in the cell culture is measured and compared to control samples. Comparison of the results, for example, will indicate test compounds that may be beneficially used in combination with Compound I or a salt thereof, or Compound I or a salt thereof plus at least one other known anti-HIV drug.

The present invention also provides a kit comprising (i) Compound I, or a pharmaceutically acceptable salt thereof, and (ii) at least one substance having anti-HIV activity other than Compound I, or a pharmaceutically acceptable salt thereof. In an embodiment of the invention, the kit comprises at least one substance having anti-HIV activity that is selected from Truvada® (tenofovir + emtricitabine), elvucitabine, GW 204937, GW 678248, MK-0518, RSC 1838, V-165, C-2507, BMS 538158, and L-900564. The kit may comprise one or more pharmaceutical compositions of the invention. The kit may further comprise a container and/or written material stating that Compound I or a salt thereof may be used for treating a HIV infection, for inhibiting HIV integrase activity, or for treating a HIV infection or inhibiting HIV integrase activity of a HIV resistant to at least one anti-HIV drug.

The present invention is illustrated by the following Examples, which are not intended to be limiting in any way.

### EXAMPLE 1

In this study, the PhenoSense™ HIV assay was used to evaluate antiviral activity of Compound I against a panel of recombinant HIV-1 isolates containing diverse *pol* proteins.

The detailed method for the PhenoSense™ HIV assay has been described in Petropoulus et al., Antimicrob. Agents Chemother. 44:920-928 (2000). Briefly, recombinant HIV-1 molecular clones were constructed by amplifying protease and RT sequences from HIV-infected patient plasma samples and inserting the amplification products into the *pol* region of the recombinant molecular clone HIV-1 NL4-3 (the reference clone). Because these clones contain a luciferase gene cassette within the env region, viral replication could be monitored by luciferase activity in the infected cells.

120 recombinant clones were produced and the mutation sites in the RT and protease regions were analyzed thoroughly for all 120 recombinant clones. Among these recombinant clones, 112 clones had one or more drug-resistance-related mutations in the protease- or RT-coding regions and eight clones did not have any crucial mutations in these regions and were therefore designated as wild-type clones.

Antiviral susceptibility to Compound I and to 16 approved anti-HIV drugs was evaluated for all 120 recombinant clones as described in Petropoulus et al., Antimicrob. Agents Chemother. 44:920-928 (2000). HEK293 cells were co-transfected in 10 cm dishes with 1) an HIV-1 genomic vector that contains the protease/reverse transcriptase region and a luciferase indicator gene cassette inserted within a deleted region of the envelope region and 2) a plasmid that expresses amphotropic murine leukemia virus envelope. For drug susceptibility evaluation, a drug sensitive resistance test vector derived from the NL4-3 laboratory strain of HIV-1 was used as the reference virus. For protease inhibitor (PI) testing, transfected cells were trypsinized approximately 24 hours after transfection and plated into 96-well plates containing serial dilutions of drugs. Viruses were harvested from the transfected HEK 293 cells the following day and were used to infect fresh HEK 293 cells in the absence of additional drugs. For reverse transcriptase (RT) and integrase inhibitor testing, virus was harvested from transfected cells in the absence of drugs approximately 48 hours after transfection. Additionally, nucleoside reverse transcriptase inhibitors (NRTIs), non-nucleoside reverse transcriptase inhibitors (NNRTIs), or Compound I were added to fresh HEK 293 cells one day prior to viral infection. The ability of the pseudovirions to infect the target cells was monitored by the production of luciferase in the infected cells approximately 72 hours after infection. The results were expressed as the ratio of the EC₅₀ value in a tested clone to that of the reference clone, NL4-3 ("fold increase").

The 112 clones with one or more drug-resistant-related mutations were categorized into three classes based on their phenotype: nucleoside reverse transcriptase inhibitor resistant (NRTI^{r}), nonnucleoside reverse transcriptase inhibitor resistant (NNRTI^{r}) and protease inhibitor resistant (PI^{r}) and are shown in Table 1. The genetic mutations associated with these phenotypes (D'Aquila et al., Topics in HIV Medicine 11:92-96, 2003) are also shown in Table 1. The genetic mutations found in the 112 clones were further categorized into 16 classes according to genotype and are shown in Table 2. Table 2 also summarizes the effect of Compound I and the 16 approved anti-HIV drugs on HIV replication of all 120 recombinant clones. The fold-increase value of each clone for each anti-HIV drug and Compound I was tabulated and averaged for each of the 16 genotype classes.

**Table 1.**

| Phenotype | Amino acid residue and position in RT region in wild type |
|---|---|
| NRTI^{r} | M41*, K65, D67*, T69, K70*, V118, Q151, M184, L210*, T215* and K219* |
| NNRTI^{r} | K103, Y181 and Y188 |

| | Amino acid residue and position in protease region in wild type |
|---|---|
| PI^{r} | D30, M46, V82, I84 and L90 |

| | |
|---|---|
| *TAMs: thymidine analogue mutations | |

**Table 2.**

| RT mutation**^{a)}** | N**^{b)}** | Fold-increase^{c)} | | | | | | | |
|---|---|---|---|---|---|---|---|---|---|
| | | Compound I | AZT | 3TC | ddI | d4T | ABC | TFV | |
| Wild-type | 8 | 0.9 | 0.8 | 1.1 | 1.1 | 1.0 | 0.9 | 0.8 | |
| 6 TAMs^{d)} | 5 | 0.9 | 531.6 | 6.7 | 2.2 | 6.2 | 6.9 | 5.0 | |
| 6 TAMs, M184 | 7 | 1.0 | 92.1 | 98.0 | 2.1 | 2.7 | 7.4 | 1.6 | |
| 2-5 TAMs^{e)} | 16 | 1.0 | 271.3 | 6.1 | 2.5 | 4.8 | 6.6 | 3.5 | |
| 2-5 TAMs, T69ins | 4 | 1.2 | 506.4 | 19.5 | 6.0 | 17.0 | 19.3 | 12.2 | |
| 2-5 TAMs, K65 and/or Q151 | 4 | 1.1 | 437.8 | 57.3 | 17.2 | 21.0 | 19.8 | 8.5 | |
| M184 | 12 | 0.9 | 0.4 | 102.4 | 1.4 | 0.8 | 3.1 | 0.6 | |
| K65 and/or Q151 | 7 | 0.9 | 181.6 | 40.1 | 9.6 | 10.3 | 13.3 | 4.0 | |
| K65 and/or Q151, M184 | 12 | 0.9 | 262.6 | 108.0 | 13.2 | 9.0 | 24.1 | 2.2 | |
| | | | | | | | | | |

| RT mutation^{a)} | N^{b)} | Fold-increase^{c)} | | | | | | | |
|---|---|---|---|---|---|---|---|---|---|
| | | Compound I | NVP | | DLV | | EFV | | |
| Wild-type | 8 | 0.9 | 1.2 | | 1.9 | | 1.3 | | |
| K103 | 46 | 0.9 | 127.3 | | 86.9 | | 128.6 | | |
| K103, Y181 | 14 | 1.0 | 187.1 | | 122.1 | | 154.7 | | |
| K103, Y188 | 6 | 0.8 | 197.0 | | 109.2 | | 218.0 | | |

| Protease mutation^{a)} | N^{b)} | Fold-increase^{c)} | | | | | | | |
|---|---|---|---|---|---|---|---|---|---|
| | | Compound I | APV | IDV | NFV | RTV | SQV | LPV | ATV |
| Wild-type | 8 | 0.9 | 0.6 | 0.7 | 0.9 | 0.8 | 0.7 | 0.7 | 0.7 |
| M46, V82 | 14 | 0.9 | 27.6 | 31.3 | 34.6 | 59.5 | 42.8 | 76.8 | 32.2 |
| M46, V82, 184 or L90 | 14 | 1.0 | 62.3 | 29.1 | 41.7 | 130.6 | 45.4 | 112.0 | 34.7 |
| M46, V82, I84, L90 | 3 | 1.0 | 34.0 | 32.0 | 64.0 | 163.3 | 108.0 | 82.3 | 45.0 |
| D30 | 8 | 1.0 | 1.1 | 1.6 | 37.8 | 1.3 | 1.3 | 1.1 | 3.7 |
| D30, L90 | 5 | 1.0 | 9.6 | 11.3 | 209.2 | 29.2 | 114.4 | 11.6 | 41.4 |

| | | | | | | | | | |
|---|---|---|---|---|---|---|---|---|---|
| Mutation sites are represented as the position number in the RT-or protease-coding region and the amino acid residue in the wild-type virus. Number of clones EC₅₀ fold increase above reference clone (NL4-3) Six thymidine analogue mutations (M41, D67, K70, L210, T215, K219) Two to 5 thymidine analogue mutations | | | | | | | | | |

Furthermore, the antiviral susceptibility of the 120 recombinant clones to Compound I are summarized in Table 3.

**Table 3.**

| | Recombinant clone | Reference clone (NL4-3) |
|---|---|---|
| Mean EC₅₀ (nM) | 1.06 | 1.11 |
| Number of | 120^{a)} clones | 1^{b)} |

| | | |
|---|---|---|
| One experiment Ten replicates, one experiment | | |

In summary, Compound I showed potent inhibitory activity against the 120 recombinant HIV clones with a mean EC₅₀ value of 1.06 nM (Table 3). This value was comparable to the EC₅₀ value for the reference clone (Table 3). Moreover, Compound I retained potent inhibitory activity against many types of drug-resistant clones, including NRTI^{r}, NNRTI^{r} and PI^{r}. In contrast, most of the 16 examined anti-HIV drugs showed significant decreases in antiviral activity against the recombinant clones carrying genetic mutations that conferred resistance to the corresponding drug (Table 2).

This study demonstrated that Compound I exhibits potent inhibitory activity against wild-type HIV-1 and various recombinant HIV clones with genetic mutations in the HIV protease and RT regions associated with drug resistance.

### EXAMPLE 2

In this study, the antiviral efficacy and cellular cytotoxicity of Compound I and other anti-retroviral drugs was evaluated in a standard peripheral blood mononuclear cell (PBMC)-based anti-HIV assay against a panel of HIV-1 subtypes, as well as against a panel of drug-resistant HIV-1 isolates. In addition, the compound was tested for antiviral efficacy in macrophages against three HIV-1 macrophage-tropic virus strains.

A 20 mM stock solution was prepared using Compound I in powder form and dimethylsulfoxide (DMSO) as the diluent. Compound I was tested at 100 nM with 8 additional serial half-log dilutions. Other anti-retroviral drugs, AZT, efavirenz, and nelfinavir were used as control compounds in all assays performed. Enfuvirtide (T-20) was also included as a control compound in the assays using drug-resistant HIV-1 isolates.

Materials. Fresh human blood was obtained commercially from Interstate Blood Bank, Inc. (Memphis, TN). 11 HIV-1 clinical isolates were chosen for this study to represent isolates from each of the seven HIV-1 Group M envelope subtypes A, B, C, D, E, F, and G as well as one isolate from HIV-1 Group O. Three additional subtype B viruses were also chosen (Table 4). All HIV-1 and HIV-2 virus isolates were obtained from the NIH AIDS Research and Reference Reagent Program. Low passage stocks of each virus were prepared using fresh human PMBCs and stored in liquid nitrogen.

The HIV-1 isolates 052-52, 1064-52, 144-44 and 1002-60 were obtained from the Merck Research Laboratories as supernatant virus. The major amino acid substitutions observed in the protease protein of these isolates is provided in Table 5. The multi-drug-resistant HIV-1 isolates MDR 769, MDR 1385, MDR 3761, and MDR 807 were obtained from Dr. Thomas C. Merigan (Stanford University). The drug resistance profile for these viruses is provided in Table 6. The virus isolates were originally derived by co-culture of primary PBMCs from each patient with normal human donor PBMCs.

**Table 4.**

| **HIV-1 Isolate** | **Gag/Env Subtype** | **Coreceptor Tropism** | **ADDITIONAL INFORMATION** |
|---|---|---|---|
| RW/92/016 | A/A | R5 | Isolated from seropositive individual in Rwanda |
| 96USHIPS7 | /B | R5 | Isolated from seronegative individual in the US |
| BR/92/021 | B/B | R5 (NSI) | Isolated from seropositive individual in Brazil |
| BR/93/017 | B/B | R5 | Isolated from seropositive individual in Brazil |
| BR/93/022 | /B | R5 | Isolated from seropositive individual in Brazil |
| BR/92/025 | C/C | R5 (NSI) | Isolated from seropositive individual in Brazil |
| UG/92/046 | D/D | X4 (SI) | Isolated from seropositive individual in Uganda |
| CMU02 | /EA | X4 (SI) | Isolated from seropositive individual in Thailand. E-A env recombinant |
| BR/93/020 | F/F | R5/X4 (SI) | Isolated from seropositive individual in Brazil |
| JV1083 | /G | R5 (NSI) | Virus obtained by cocultivation of PBMCs from a Nigerian AIDS patient with normal, uninfected PBMCs |
| BCF01 | Group O | R5 (NSI) | Isolated from a symptomatic, 44-year-old man from Cameroon |

**Table 5.**

| Isolate | Protease Mutations |
|---|---|
| 1064-52 | L10I / I54V / L63P / A71T / V82F / L90M |
| 1002-60 | L10I / M46I / I54V / L63P / V82F / L90M |
| 144-44 | V32I / M46I / L63P / L90M |
| 052-52 | L10R / M46I / L63P / A71V / V82T / I84V |

**Table 6.**

| Virus | Gene | Resistance Mutations | Other Changes from Consensus B | Drug Resistance |
|---|---|---|---|---|
| MDR 769 | RT | M41L, K65R, D67N, V75I, F116Y, Q151M, Y181I, L210W, T215Y | K20R, V21I, V35I, K43Q, A62V, E79D, I167I/V, G196E, Q197K, E207Q, D218E | AZT, ddI, 3TC, d4T, PFA, NVP |
| MDR769 | PR | L10I, M36M/V, M46I, 154V, L63P, A71V, V82A, I84V, L90M | V13I, D60E, 162V, K223Q | IDV, SQV, NFV |
| MDR 3761 | PR | L10I, M46I, I84V, L63P, A71I, L90M | T12A, V13I, I15V, L19I, K20I, I64P,G73T, P79S, I93L | AZT, ddI, 3TC, d4T, NVP, IDV, SQV, NFV |
| MDR 1385 | PR | L10I, M36I, M46I, I54I/V, L63P, A71V, V82T, L90M | V13I, N37D, R57K, L89M, I93L | AZT, ddI, 3TC, d4T, IDV, SQV |
| MDR 807 | RT | M41L, D67N, M184V, L210W, T215Y, K219N | K43N, V60I, A98S, V118I, I135V, G196E, R211K | AZT, ddI, 3TC, d4T |
| MDR 807 | PR | L10I, G48V, I54T, L63Q, A71V, V82A | V13I, R41K, I62V, T74A, V77I, I93L | IDV, SQV, NFV |

| | | | | |
|---|---|---|---|---|
| Mutations in bold face type represent key resistance mutations in the indicated genes. | | | | |

In all assays performed, pre-titered aliquots of each virus were removed from the freezer (-80°C) and thawed rapidly to room temperature in a biological safety cabinet immediately before use. Phytohemagglutinin (PHA-P) was obtained from Sigma (St. Louis, MO) and recombinant IL-2 was obtained from R&D Systems Inc. (Minneapolis, MN).

Anti-HIV Efficacy Evaluation in Fresh Human PBMCs. Fresh human PBMCs, seronegative for HIV and hepatitis B virus (HBV), were isolated from screened donors (Interstate Blood Bank, Inc. Memphis, TN). Cells were pelleted/washed 2-3 times by low speed centrifugation and resuspended in PBS to remove contaminating platelets. The leukophoresed blood was then diluted 1:1 with Dulbecco's Phosphate Buffered Saline (DPBS) and layered over 14 mL of Lymphocyte Separation Medium (LSM; Cellgro® by Mediatech, Inc.; density 1.078+/-0.002 g/ml; Cat.# 85-072-CL) in a 50 mL centrifuge tube and then centrifuged for 30 minutes at 600 x g. Banded PBMCs were gently aspirated from the resulting interface and subsequently washed two times with PBS by low speed centrifugation. After the final wash, cells were counted by trypan blue exclusion and resuspended at 1 x 10⁷ cells/mL in RPMI 1640 supplemented with 15% Fetal Bovine Serum (FBS), and 2 mM L-glutamine, 4 µg/mL Phytohemagglutinin (PHA-P, Sigma). The cells were allowed to incubate for 48-72 hours at 37°C. After incubation, PBMCs were centrifuged and resuspended in RPMI 1640 with 15% FBS, 2 mM L-glutamine, 100 U/mL penicillin, 100 µg/mL streptomycin, 10 µg/mL gentamycin, and 20 U/mL recombinant human IL-2 (R&D Systems, Inc). IL-2 was included in the culture medium to maintain the cell division initiated by the PHA mitogenic stimulation. PBMCs were maintained in this medium at a concentration of 1-2 x 10⁶ cells/mL with biweekly medium changes until used in the assay protocol. Cells were kept in culture for a maximum of two weeks before being deemed too old for use in assays and discarded. Monocytes were depleted from the culture as the result of adherence to the tissue culture flask.

For the standard PBMC assay, PHA-P stimulated cells from at least two normal donors were pooled (mixed together), diluted in fresh medium to a final concentration of 1 x 10⁶ cells/mL, and plated in the interior wells of a 96 well round bottom microplate at 50 µL/well (5 x 10⁴ cells/well) in a standard format developed by the Infectious Disease Research department of Southern Research Institute. Pooling (mixing) of mononuclear cells from more than one donor was used to minimize the variability observed between individual donors, which results from quantitative and qualitative differences in HIV infection and overall response to the PHA and IL-2 of primary lymphocyte populations. Each plate contained virus/cell control wells (cells plus virus), experimental wells (drug plus cells plus virus) and compound control wells (drug plus media without cells, necessary for MTS monitoring of cytotoxicity described below). Since HIV-1 is not cytopathic to PBMCs, this allows the use of the same assay plate for both antiviral activity and cytotoxicity measurements.

Test drug dilutions were prepared at a 2X concentration in microtiter tubes and 100 µL of each concentration was placed in appropriate wells using the standard format. 50 µL of a predetermined dilution of virus stock was placed in each test well (final multiplicity of infection (MOI) ≅ 0.1). The PBMC cultures were maintained for seven days following infection at 37°C, 5% CO₂. After this period, cell-free supernatant samples were collected for analysis of reverse transcriptase activity and p24 antigen ELISA. Following removal of supernatant samples, cytotoxicity was measured by addition of 3-(4,5-dimethylthiazol-2-yl)-5-(3-carboxymethoxyphenyl)-2-(4-sulfophenyl)-2H-tetrazolium, inner salt (MTS) to the plates for determination of cell viability. Wells were also examined microscopically and any abnormalities were noted.

For certain virus isolates (BR/92/025, BCF01, and Merck 144-44), the standard assay was modified slightly to include a pre-incubation step of virus with the PBMCs prior to the addition of drug in order to obtain adequate and more consistent levels of virus infection in the cultures. In the modified assays, 8 x 10⁶ cells from pooled donors were incubated with 1.0 mL of stock virus for one hour at 37°C, 5% CO₂, followed by centrifugation for one hour at 900 rpm (200 x g). Cells were then gently resuspended and incubated an additional two hours. During this second incubation period, 2.5 x 10⁴ uninfected PBMCs from pooled donors were added to the interior 60 wells of a round-bottom 96-well plate in a volume of 50 µL of media, followed by the addition of 100 µL of diluted compounds in media at 2X concentration to the appropriate wells. At the end of the second incubation period, infected cells were diluted in media to a concentration of 5 x 10⁵ cells/mL (without washout of virus) and 50 µL (2.5 x 10⁴ cells) were added to each well of the plate. The remaining steps of the assay were performed in the same manner as for the standard assay.

### Anti-HIV Efficacy Evaluation in Fresh Human

Monocyte/Macrophage. Monocytes/macrophages were allowed to adhere to the interior 60 wells of a 96 well flat bottomed plate for 2 to 18 hours at 37°C, 5% CO₂. Following 6 to 14 days in culture, the monocyte/macrophage cultures were washed 3 times to remove any non-adherent cells and serially diluted test compounds were added followed by the addition of a pre-titered amount of HIV. Cultures were washed a final time by media removal 24 hours post infection, fresh compound added and the cultures continued for an additional six days. The assays were performed using the standardized microtiter plate format developed by the Infectious Disease Research department of Southern Research Institute and described above. At assay termination, virus replication was measured by collecting cell-free supernatant samples which were analyzed for HIV p24 antigen content using a commercially available p24 ELISA assay (Coulter). Following removal of supernatant samples, compound cytotoxicity was measured by addition of MTS to the plates for determination of cell viability. Wells were also examined microscopically and any abnormalities noted. The HIV reverse transcriptase inhibitor AZT was used as a positive control compound and run in parallel with each determination.

Reverse transcriptase activity assay. A microtiter plate-based reverse transcriptase (RT) reaction was utilized (Buckheit et al., AIDS Research and Human Retroviruses 7:295-302, 1991). Tritiated thymidine triphosphate (³H-TTP, 80 Ci/mmol, NEN) was received in 1:1 dH₂O:Ethanol at 1 mCi/mL. Poly rA:oligo dT template:primer (Pharmacia) was prepared as a stock solution by combining 150 µL poly rA (20 mg/mL) with 0.5 mL oligo dT (20 units/mL) and 5.35 mL sterile dH₂O followed by aliquoting (1.0 mL) and storage at -20°C. The RT reaction buffer was prepared fresh on a daily basis and consisted of 125 µL 1.0 M EGTA, 125 µL dH₂O, 125 µL 20% Triton X100, 50 µL 1.0 M Tris (pH 7.4), 50 µL 1.0 M DTT, and 40 µL 1.0 M MgCl₂. The final reaction mixture was prepared by combining 1 part ³H-TTP, 4 parts dH₂O, 2.5 parts poly rA:oligo dT stock and 2.5 parts reaction buffer. Ten microliters of this reaction mixture was placed in a round bottom microtiter plate and 15 µL of virus containing supernatant was added and mixed. The plate was incubated at 37°C for 60 minutes. Following incubation, the reaction volume was spotted onto DE81 filter-mats (Wallach), washed 5 times for 5 minutes each in a 5% sodium phosphate buffer or 2X SSC (Life Technologies). Next they were washed 2 times for 1 minute each in distilled water, 2 times for 1 minute each in 70% ethanol, and then dried. Incorporated radioactivity (counts per minute, CPM) was quantified using standard liquid scintillation techniques.

### MTS staining for PBMC viability to measure cytotoxicity.

At assay termination, assay plates were stained with the soluble tetrazolium-based dye MTS (3-(4,5-dimethylthiazol-2-yl)-5-(3-carboxymethoxyphenyl)-2-(4-sulfophenyl)-2H-tetrazolium, inner salt) (CellTiter 96 Reagent, Promega) to determine cell viability and to quantify compound toxicity. The mitochondrial enzymes of metabolically active cells metabolize MTS to yield a soluble formazan product. This allows the rapid quantitative analysis of cell viability and compound cytotoxicity. The MTS is a stable solution that does not require preparation before use. At termination of the assay, 20 µL of MTS reagent was added per well. The microtiter plates were then incubated 4-6 hrs at 37°C. The incubation intervals were chosen based on empirically determined times for optimal dye reduction. Adhesive plate sealers were used in place of the lids, the sealed plate was inverted several times to mix the soluble formazan product and the plate was read spectrophotometrically at 490/650 nm with a Molecular Devices Vmax or SpectraMaxPlus plate reader.

p24 Antigen ELISA. ELISA kits were purchased from Coulter Electronics. The assay was performed according to the manufacturer's instructions. Control curves were generated in each assay to accurately quantify the amount of p24 antigen in each sample. Data were obtained by spectrophotometric analysis at 450 nm using a Molecular Devices Vmax plate reader. Final concentrations were calculated from the optical density values using the Molecular Devices SOFTmax Pro software package.

Data analysis. Using an in-house computer program, IC ₅₀ (50%, inhibition of virus replication), TC₅₀ (50% cytotoxicity) and therapeutic index (TI, TC₅₀/IC₅₀) were determined for each virus and compound and are shown in Tables 7-10.

**Table 7. Antiviral Efficacy Against HIV-1 Viruses in PBMC**

| Virus | Compound | IC₅₀ (nM) | TC₅₀ (nM) | TI |
|---|---|---|---|---|
| RW/92/016 | Compound I | 0.41 | > 100.0 | > 243.9 |
| | AZT | 7.91 | > 1000.0 | > 126.4 |
| | Efavirenz | 0.61 | > 1000.0 | > 1639.3 |
| | Nelfinavir | 13.40 | > 1000.0 | > 74.6 |
| 96USHIPS7 | Compound I | 0.26 | > 100.0 | > 384.6 |
| | AZT | 8.41 | > 1000.0 | > 118.9 |
| | Efavirenz | 0.65 | > 1000.0 | > 1538.5 |
| | Nelfinavir | 25.8 | > 1000.0 | > 38.8 |
| BR/92/021 | Compound I | 0.76 | > 100.0 | > 131.6 |
| | AZT | 2.13 | > 1000.0 | > 469.5 |
| | Efavirenz | 47.0 | > 1000.0 | > 21.3 |
| | Nelfinavir | 70.5 | > 1000.0 | > 14.2 |
| BR/93/017 | Compound I | 0.18 | > 100.0 | > 555.6 |
| | AZT | 1.10 | > 1000.0 | > 909.1 |
| | Efavirenz | 0.58 | > 1000.0 | > 1724.1 |
| | Nelfinavir | 2.05 | > 1000.0 | > 487.8 |
| BR/93/022 | Compound I | 1.13 | > 100.0 | > 88.5 |
| | AZT | 11.7 | > 1000.0 | > 85.5 |
| | Efavirenz | 0.62 | > 1000.0 | > 1612.9 |
| | Nelfinavir | 20.1 | > 1000.0 | > 49.8 |
| BR/92/025 | Compound I | 0.10 | > 100.0 | > 1000.0 |
| | AZT | 2.84 | > 1000.0 | > 352.1 |
| | Efavirenz | 0.30 | > 1000.0 | > 3333.3 |
| | Nelfinavir | < 0.10 | > 1000.0 | > 9901.0 |
| UG/92/045 | Compound I | 0.50 | > 100.0 | > 200.0 |
| | AZT | 7.26 | > 1000.0 | > 137.7 |
| | Efavirenz | 1.19 | > 1000.0 | > 840.3 |
| | Nelfinavir | 27.7 | > 1000.0 | > 36.1 |
| CMU 02 | Compound I | 1.26 | > 100.0 | > 79.4 |
| | AZT | 9.07 | > 1000.0 | > 110.3 |
| | Efavirenz | 1.82 | > 1000.0 | > 549.5 |
| | Nelfinavir | 22.7 | > 1000.0 | > 44.1 |
| BR/93/020 | Compound I | 0.74 | > 100.0 | > 135.1 |
| | AZT | 25.3 | > 1000.0 | > 39.5 |
| | Efavirenz | 0.32 | > 1000.0 | > 3125.0 |
| | Nelfinavir | 16.4 | > 1000.0 | > 61.0 |
| JV 1083 | Compound I | 0.35 | > 100.0 | > 285.7 |
| | AZT | 11.1 | > 1000.0 | > 90.1 |
| | Efavirenz | 0.65 | > 1000.0 | > 1538.5 |
| | Nelfinavir | 14.0 | > 1000.0 | > 71.4 |
| BFC01 | Compound I | 1.17 | > 100.0 | > 85.5 |
| | AZT | 1.52 | > 1000.0 | > 657.9 |
| | Efavirenz | 0.69 | > 1000.0 | > 1449.3 |
| | Nelfinavir | 0.99 | > 1000.0 | > 1010.1 |

**Table 8. Antiviral Efficacy Against HIV-1 Drug-Resistant Viruses in PBMCs**

| Virus | Compound | IC₅₀ (nM) | TC₅₀ (nM) | TI |
|---|---|---|---|---|
| 1064-52 | Compound I | 0.63 | > 100.0 | > 158.7 |
| | AZT | 13.6 | > 1000.0 | > 73.5 |
| | Efavirenz | 0.29 | > 1000.0 | > 3448.3 |
| | Nelfinavir | 838.5 | > 1000.0 | > 1.19 |
| | T-20 | 275.5 | > 10000.0 | > 36.3 |
| 52-52 | Compound I | 0.17 | > 100.0 | > 588.2 |
| | AZT | 63.9 | > 1000.0 | > 15.7 |
| | Efavirenz | < 0.10 | > 1000.0 | > 9901.0 |
| | Nelfinavir | 95.0 | > 1000.0 | > 10.5 |
| | T-20 | 1.81 | > 10000.0 | > 5524.9 |
| 1002-60 | Compound I | 0.13 | > 100.0 | > 769.2 |
| | AZT | 6.72 | > 1000.0 | > 148.8 |
| | Efavirenz | 0.21 | > 1000.0 | > 4761.9 |
| | Nelfinavir | > 1000.0 | > 1000.0 | N/A |
| | T-20 | 2.86 | > 10000.0 | > 3496.5 |
| 144-44 | Compound I | 0.72 | > 100.0 | > 138.89 |
| | AZT | 7.24 | > 1000.0 | > 138.1 |
| | Efavirenz | 0.60 | > 1000.0 | > 1666.7 |
| | Nelfinavir | 165.3 | > 1000.0 | > 6.05 |
| | T-20 | 635.9 | > 10000.0 | > 15.7 |
| MDR 769 | Compound I | 0.18 | > 100.0 | > 555,6 |
| | AZT | 720.7 | > 1000.0 | > 1.39 |
| | Efavirenz | 0.54 | > 1000.0 | > 1851.9 |
| | Nelfinavir | > 1000.0 | > 1000.0 | N/A |
| | T-20 | 86.5 | > 10000.0 | > 115.6 |
| MDR 1385 | Compound I | 0.02 | > 100.0 | > 5000.0 |
| | AZT | > 1000.0 | > 1000.0 | N/A |
| | Efavirenz | > 1000.0 | > 1000.0 | N/A |
| | Nelfinavir | > 1000.0 | > 1000.0 | N/A |
| | T-20 | 16.8 | > 10000.0 | > 595.2 |
| MDR 3761 | Compound I | 0.73 | > 100.0 | > 137.0 |
| | AZT | > 1000.0 | > 1000.0 | N/A |
| | Efavirenz | > 1000.0 | > 1000.0 | N/A |
| | Nelfinavir | > 1000.0 | > 1000.0 | N/A |
| | T-20 | 46.5 | > 10000.0 | > 215.1 |
| MDR 807 | Compound I | 1.13 | > 100.0 | > 88.5 |
| | AZT | 159.4 | > 1000.0 | > 6.27 |
| | Efavirenz | 0.71 | > 1000.0 | > 1408.5 |
| | Nelfinavir | 135.0 | > 1000.0 | > 7.41 |
| | T-20 | 3147.4 | > 10000.0 | > 3.18 |

**Table 9. Antiviral Efficacy Against HIV-1 Viruses in Macrophages**

| Virus | Compound | IC₅₀ (nM) | TC₅₀ (nM) | TI |
|---|---|---|---|---|
| Ba-L | Compound I | 0.67 | > 500.0 | > 746.3 |
| | AZT | 0.20 | > 1000.0 | > 5000.0 |
| | Efavirenz | 2.12 | > 10000.0 | > 4717.0 |
| | Nelfinavir | 36.3 | > 10000.0 | > 275.5 |
| ADA | Compound I | 0.07 | > 500.0 | > 7142.9 |
| | AZT | 0.18 | > 1000.0 | > 5555.6 |
| | Efavirenz | 3.58 | > 10000.0 | > 2793.3 |
| | Nelfinavir | 45.2 | > 10000.0 | > 221.2 |
| JR-CSF | Compound I | 0.31 | > 100.0 | >322.6 |
| | AZT | 3.82 | > 1000.0 | >261.8 |

**Table 10. Antiviral Efficacy Against HIV-2 Viruses in PBMCs**

| Virus | Compound | IC₅₀ (nM) | TC₅₀ (nM) | TI |
|---|---|---|---|---|
| CDC 310319 | Compound I | 0.53 | > 100.0 | > 188.7 |
| | AZT | 1.14 | > 1000.0 | > 877.2 |
| | Efavirenz | > 1000.0 | > 1000.0 | N/A |
| | Nelfinavir | 16.1 | > 1000.0 | > 62.1 |

As shown in the results summarized in Tables 7-10, Compound I was found to be a highly active anti-HIV compound with broad activity against all HIV-1, HIV-2 and drug-resistant isolates tested in this study. Compound I was highly potent against viruses with a drug-sensitive phenotype (i.e., wild-type viruses; Tables 7 and 9), with average IC₅₀ values of 0.62 ± 0.41 nM in PBMCs (n = 11 viruses) and 0.35 ± 0.30 nM in macrophage cultures (n = 3 viruses), respectively. Similar antiviral potency (IC50 = 0.53 nM) was also observed against a single HIV-2 isolate (Table 10). Compared to the other FDA-approved reverse transcriptase and protease inhibitors evaluated in this study (i.e., AZT, efavirenz, and nelfinavir), Compound I was the most potent compound evaluated. Efavirenz and Compound I had similar potency against most of the drug-sensitive viruses that were evaluated in this study (Tables 7 and 9). Compound I had greater or equivalent potency compared to all control compounds against the multi-drug resistant viruses evaluated in this study (Table 8).

Compound I also retained potent antiviral activity in PBMCs against all the viruses tested in the drug-resistant virus panel (Table 8). Utilizing viruses with well-characterized protease drug-resistant and multidrug-resistant phenotypes, Compound I retained full antiviral potency with average IC₅₀ values of 0.46 ± 0.39 nM in PBMCs (n = 8 viruses). Against several viruses (e.g., MDR 1385, MDR 3761), Compound I retained antiviral efficacy (IC₅₀ < 1 nM) while AZT, efavirenz, and nelfinavir were completely inactive (IC₅₀ > 1000 nM) against these viruses.

The overall performance of the assays was validated by the positive control compounds exhibiting the expected level of antiviral activity. Furthermore, the MOI-sensitive positive control compound AZT yielded expected results (IC₅₀ <10 nM). This provides evidence that proper virus titers were utilized in the reported antiviral assays. Macroscopic observation of the cells in each well of the microtiter plate confirmed the cytotoxicity results obtained following staining of the cells with the MTS metabolic dye.

### EXAMPLE 3

The effect of the combined use of Compound I with known anti-HIV agents may be determined as described below.

The effect of a combination of Compound I with at least one anti-viral drug, such as a nucleoside reverse transcriptase inhibitor (e.g., Zidovudine, Lamivudine, Tenofovir), a non-nucleoside reverse transcriptase inhibitor (e.g., Efavirenz), a protease inhibitor (e.g., Indinavir, Nelfinavir), or an integrase inhibitor, may be evaluated in an acute infection system using HIV-1 IIIB-infected CEM-SS cells by the XTT method (Weislow et al., J. Natl. Cancer Inst. 81:577-586, 1989; Roehm et al., J. Immunol. Methods 142:257-265, 1991). The effect of a combination of Compound I with at least two anti-retroviral drugs may also be evaluated using this system.

First, the IC₅₀ and CC₅₀ (50% cytotoxicity dose) of each anti-retroviral drug alone are determined. Then, compositions comprising various concentrations of Compound I and various concentrations of the other anti-retroviral drugs are prepared and evaluated. In combinations comprising Compound I and at least two anti-viral agents, the anti-viral agents other than Compound I are mixed and combined with various concentrations of Compound I and evaluated.

The experimental data are analyzed using the programs of Prichard and Shipman MacSynergy II version 2.01 and Delta graph version 1.5 d. A three dimensional plot is created at a 95% (or 68%, 99%) confidence level, from the percent inhibition at the concentration of each combined anti-retroviral drug obtained from triplicate experiments, and the effect of combined use is evaluated based on the numerical values of µM²% calculated therefrom. The evaluation criteria are shown in the following.

| **Definition of Interaction** | **µM²%** |
|---|---|
| Highly synergistic | >100 |
| Slightly synergistic | +51 to +100 |
| Additive | +50 to -50 |
| Slightly antagonistic | -51 to -100 |
| Highly antagonistic | <-100 |

All references, including publications, patent applications, and patents, cited herein are hereby incorporated by reference in their entirety.

The use of the terms "a" and "an" and "the" and similar referents in the context of describing the invention (including the following claims) are to be construed to cover both the singular and the plural, unless otherwise indicated herein or clearly contradicted by context. The terms "comprising", "having", "including", and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein may be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The embodiments within the specification provide an illustration of embodiments of the invention and should not be construed to limit the scope of the invention. The skilled artisan recognizes that many other embodiments are encompassed by the claimed invention and that it is intended that the specification and examples be considered as exemplary only, with the true scope and spirit of the invention being indicated by the following claims.

This application is based on U.S. Provisional application No. 60/763,900 filed in the United States, the contents of which are hereby incorporated by reference. All of the references cited herein, including patents, patent applications and publications, are hereby incorporated in their entireties by reference.

## Claims

1. Use of a therapeutically effective amount of (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, for the production of an agent for the treatment of a human immunodeficiency virus (HIV) infection in a patient, wherein the HIV is resistant to at least one anti-HIV drug.

2. The use of claim 1, wherein the at least one anti-HIV drug is selected from a protease inhibitor and a reverse transcriptase inhibitor.

3. The use of claim 2, wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).

4. The use of claim 2, wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).

5. The use of claim 1, wherein the agent is orally administrated to a patient.

6. Use of a therapeutically effective amount of (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, for the production of an agent for the treatment of a human immunodeficiency virus (HIV) infection in a patient, wherein the patient has a resistance to at least one anti-HIV drug.

7. The use of claim 6, wherein the at least one anti-HIV drug is selected from a protease inhibitor and a reverse transcriptase inhibitor.

8. The use of claim 7, wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).

9. The use of claim 7, wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).

10. The use of claim 6, wherein the agent is orally administrated to a patient.

11. Use of a therapeutically effective amount of (i) 6-(3-chloro-2-fluorobenzyl)-1-((*2S*)-1-hydroxy-3-methylbutan-2-yl)-7-methoxy-4-oxo-1,4-dihydroquinoline-3-carboxylic acid, or a pharmaceutically acceptable salt thereof, and (ii) at least one substance having anti-HIV activity other than (i), for the production of an agent for the treatment of a HIV infection comprising in a patient, wherein the HIV is resistant to at least one anti-HIV drug.

12. The use of claim 11, wherein the at least one anti-HIV drug and the at least one substance having anti-HIV activity are the same.

13. The use of claim 11, wherein the at least one anti-HIV drug and the at least one substance having anti-HIV activity are different.

14. The use of claim 11, wherein the at least one anti-HIV drug is selected from a protease inhibitor and a reverse transcriptase inhibitor.

15. The use of claim 11, wherein the at least one substance having anti-HIV activity is selected from a protease inhibitor and a reverse transcriptase inhibitor.

16. The use of claim 14 or 15, wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).

17. The use of claim 14 or 15, wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).

18. The use of claim 11, wherein (i) and (ii) are orally administrated to a patient.

19. The use of claim 11, wherein (i) and (ii) are simultaneously administrated to a patient.

20. The use of claim 11, wherein (i) and (ii) are sequentially administrated to a patient.

21. Use of a therapeutically effective amount of (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, for the production of an agent for the inhibition of HIV integrase activity in a patient, wherein the HIV is resistant to at least one anti-HIV drug.

22. The use of claim 21, wherein the at least one anti-HIV drug is selected from a protease inhibitor and a reverse transcriptase inhibitor.

23. The use of claim 22, wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV), Prozei^{®} (amprenavir or APV), and Reyataz^{®} (atazanavir or ATV).

24. The use of claim 22, wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Virarnune^{®} (nevirapine or NVP), Stocrin^{®} (efavirenz or EFV), Rescriptor^{®} (delavirdine mesylate or DLV), and Tenofovir (PMPA or TFV).

25. Use of a therapeutically effective amount of (i) 6-(3-chloro-2-fluorobenzyl)-1-[(2S)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), for the production of an agent for the inhibition of HIV integrase activity in a patient, wherein the HIV is resistant to at least one anti-HIV drug.

26. The use of claim 25, wherein the at least one anti-HIV drug and the at least one substance having anti-HIV activity are the same.

27. The use of claim 25, wherein the at least one anti-HIV drug and the at least one substance having anti-HIV activity are different.

28. The use of claim 25, wherein the at least one anti-HIV drug is selected from a protease inhibitor and a reverse transcriptase inhibitor.

29. The use of claim 25, wherein the at least one substance having anti-HIV activity is selected from a protease inhibitor and a reverse transcriptase inhibitor.

30. The use of claim 28 or 29, wherein the protease inhibitor is selected from Crixivan^{®} (indinavir sulfate ethanolate or IDV), saquinavir, Invirase^{®} (saquinavir mesylate or SQV), Norvir^{®} (ritonavir or RTV), Viracept^{®} (nelfinavir mesylate or NFV), lopinavir (LPV) , Prozei^{®} (amprenavir or APV), and Reyataz^{®} (atazanavir or ATV).

31. The use of claim 28 or 29, wherein the reverse transcriptase inhibitor is selected from Retrovir^{®} (zidovudine or AZT), Epivir^{®} (lamivudine or 3TC), Zerit^{®} (sanilvudine or d4T), Videx^{®} (didanosine or ddI), Ziagen^{®} (abacavir sulfate or ABC), Viramune^{®} (nevirapine or NVP), Stocrin^{®} (efavirenz or EFV), Rescriptor^{®} (delavirdine mesylate or DLV), and Tenofovir (PMPA or TFV).

32. The use of claim 25, wherein (i) and (ii) are orally administrated to a patient.

33. The use of claim 25, wherein (i) and (ii) are simultaneously administrated to a patient.

34. The use of claim 25, wherein (i) and (ii) are sequentially administrated to a patient.

35. Use of (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, for inhibiting HIV integrase activity, comprising contacting an effective amount of (i) with a HIV resistant to at least one anti-HIV drug.

36. The use of claim 35, wherein the at least one anti-HIV drug is selected from a protease inhibitor and a reverse transcriptase inhibitor.

37. Use of a therapeutically effective amount of (i) 6-(3-chloro-2-fluorobenzyl)-1-[(*2S*)-1-hydroxy-3-methylbutan-2-yl]-7-methoxy-4-oxo-1,4-dihydroquinaline-3-carboxylic acid, or a pharmaceutically acceptable salt thereof, and (ii) at least one substance having anti-HIV activity other than (i), for the production of an agent for the treatment of a HIV infection to a patient, wherein the at least one substance having anti-HIV activity is selected from Truvada® (tenofovir + emtricitabine), elvucitabine, GW 204937, GW 678248, MK-0518, RSC 1838, V-165, C-2507, BMS 538158, and L-900564.

38. The use of claim 37, wherein (i) and (ii) are orally administrated to a patient.

39. The use of claim 37, wherein (i) and (ii) are simultaneously administrated to a patient.

40. The use of claim 37, wherein (i) and (ii) are sequentially administrated to a patient.

41. A pharmaceutical composition comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, (ii) at least one substance having anti-HIV activity is selected from Truvada® (tenofovir + emtricitabine), elvucitabine, GW 204937, GW 678248, MK-0518, RSC 1838, V-165, C-2507, BMS 538158, and L-900564; and (iii) a pharmaceutically acceptable carrier.

42. A kit comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity is selected from Truvada® (tenofovir + emtricitabine), elvucitabine, GW 204937, GW 678248, MK-0518, RSC 1838, V-165, C-2507, BMS 538158, and L-900564.

43. A kit comprising the pharmaceutical composition of claim 41.

44. The kit of claim 42, wherein (i) and (ii) are simultaneously administrated to a patient.

45. The kit of claim 42, wherein (i) and (ii) are sequentially administrated to a patient.

46. An anti-HIV agent comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, wherein the HIV is resistant to at least one anti-HIV drug.

47. An anti-HIV agent comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, wherein a patient to whom is administrated the agent has a resistance to at least one anti-HIV drug.

48. An anti-HIV agent comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), wherein the HIV is resistant to at least one anti-HIV drug.

49. A HIV integrase inhibitor comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, wherein the HIV is resistant to at least one anti-HIV drug.

50. A HIV integrase inhibitor comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), wherein the HIV is resistant to at least one anti-HIV drug.

51. A HIV integrase inhibitor comprising (i) 6-(3-chloro-2-fluorobenzyl)-1-[(*2S*)-1-hydroxy-3-methylbutan-2-yl]-7-methoxy-4-oxo-1,4-dihydroquinoline-3-carboxylic acid, or a pharmaceutically acceptable salt thereof, in combination with a HIV resistant, wherein HIV is resistant to at least one anti-HIV drug.

52. An anti-HIV agent comprising (i) 6-(3-chloro-2-fluorobenzyl)-1-[(25)-1-hydroxy-3-methylbutan-2-yl]-7-methoxy-4-oxo-1,4-dihydroquinoline-3-carboxylic acid, or a pharmaceutically acceptable salt thereof, and (ii) at least one substance having anti-HIV activity other than (i), wherein at least one substance is selected from Truvada® (tenofovir + emtricitabine), elvucitabine, GW 204937, GW 678248, MK-0518, RSC 1838, V-165, C-2507, BMS 538158, and L-900564; and (iii) a pharmaceutically acceptable carrier.
